(19) **Europäisches Patentamt**

**European Patent Office**

**Office européen des brevets**

(11) **EP 0 770 406 B1**

(12) **EUROPEAN PATENT SPECIFICATION**

(45) Date of publication and mention
of the grant of the patent:
**07.05.2003 Bulletin 2003/19**

(51) Int Cl.⁷: **A61N 1/365**, A61N 1/37

(21) Application number: **96116753.3**

(22) Date of filing: **18.10.1996**

(54) **Cardiac pacemaker**

Herzschrittmacher

Stimulateur cardiaque

(84) Designated Contracting States:
**AT BE CH DE DK ES FI FR GB GR IE IT LI LU MC NL PT SE**
Designated Extension States:
**AL LT LV SI**

(30) Priority: **20.10.1995 IT TO950853**

(43) Date of publication of application:
**02.05.1997 Bulletin 1997/18**

(73) Proprietor: **SORIN BIOMEDICA CRM S.r.l.**
**13040 Saluggia (Vercelli) (IT)**

(72) Inventors:
• **Plicchi, Gianni**
**40136 Bologna (IT)**
• **Corbucci, Giorgio**
**40017 San Giovanni in Persiceto, Bologna (IT)**
• **Garberoglio, Bruno**
**10156 Torino (IT)**

(74) Representative: **Bosotti, Luciano et al**
**c/o Buzzi, Notaro & Antonielli d'Oulx**
**Via Maria Vittoria 18**
**10123 Torino (IT)**

(56) References cited:
**EP-A- 0 443 495**      **EP-A- 0 655 260**
**US-A- 5 423 867**      **US-A- 5 549 650**

EP 0 770 406 B1

**Description**

The present invention generally relates to improvements in the solution disclosed in EP-A-0 655 260, after which the preamble of claim 1 was patterned.

In such previous application determination of the myocardial function is disclosed by means of accelerometric means sensitive, in use, to the so-called Natural Heart Acceleration (or, briefly, NHA), with the provisions of control and processing means adapted for suitably isolating at least one segment of the accelerometric signal corresponding to NHA.

The present invention, having the features called for in the following claims, specifically relates to a stimulator which constitutes an improvement of such previous solutions.

The invention will now be described, just by way of examples, with reference to the annexed drawings, where:

- figures 1 and 2 disclose, in a schematic way, the general situation of use of a device according to the invention,
- figures 3a and 3b are respective sets of time diagrams relating to experimentally developed detection signals of cardiac activity;
- figure 4 is another diagram essentially disclosing an algorithm for controlling the stimulation frequency as a function of the peak endocardial acceleration (PEA);
- figure 5 discloses a possible variant to the solution referred to in figure 4; and
- figures 6a and 6b are two further diagrams showing how the response curve of a device according to the invention can be adapted to the needs of a patient.

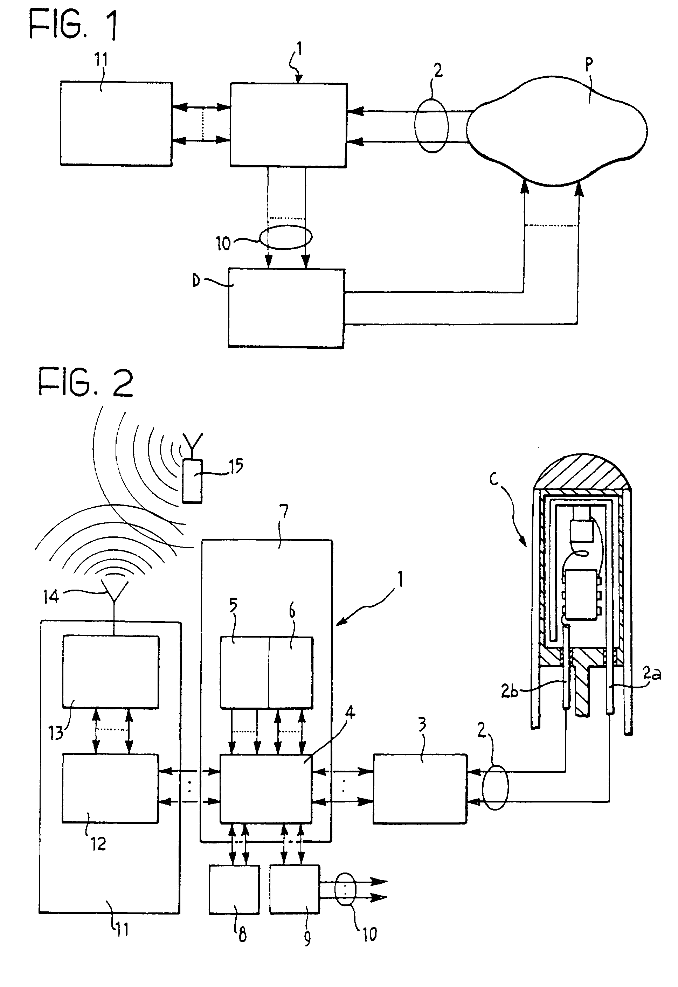

A therapeutic device utilizing the NHA signal segments is a stimulator (such as a pace-maker) of the type currently designated rate responsive. The stimulator may be either a single or dual chamber pacer. Such a stimulator would include a pulse generator, an acceleration sensor, an electrocardiogram (ECG) sensor and appropriate pacing electrodes for providing stimulation pulses to the atrium, the ventricle or both, depending on the patients' needs. The features of such a pacemaker have been generally discussed in the above captioned applications, specifically in connection with Figs. 1 and 2, which are reproduced herewith the same numbering. The NHA signal is processed to calculate the peak-to-peak value. The peak-to-peak value as measured inside a time window containing an isolated phase of the cardiac cycle,such as theisovolumetric contraction phase, is identified as the Peak Endocardial Acceleration (or, briefly, PEA, which designation is reproduced also in the drawings annexed to the present description).

The PEA has been found in normal hearts to have excellent correlation with both dP/dt maximum of the right ventricle (RV) and dP/dt maximum of the left ventricle (LV). RV dP/dt maximum has been previously used in the field of cardiac pacing for driving the pacing rate in rate responsive pacemakers. However, the contractile function of the whole heart is largely dependent on the left ventricle where most of the muscular mass is found. There are some conditions of the heart where LV dP/dt maximum does not correlate with RV dP/dt maximum. Thus, during these conditions RV dP/dt maximum does not provide a reliable indication of the contractile function of the heart.

In Figures 1 and 2, which, as indicated, reproduce Figures 1 and 2 of previous application EP-A-0 655 260, the block 1 generally indicates a device according to the invention intended to detect the myocardiac acceleration in at least one isovolumic phase and to process parameters derived from this; this device is intended to be implanted in the body of a patient P.

The block 11 generally indicates a device for the bidirectional transmission of data between the interior of the patient's body and the outside for the purpose of monitoring the myocardial acceleration and parameters derived from cardiac function and for programming the operation of block 1.

The blocks 1 and 11 are desirably integrated in a single implantable device and are intended to operate together with the therapeutic device D which is preferably also implantable and preferably integrated with the device constituted by the blocks 1 and 11. The device D is intended to act on the patient P in an electrical manner. Therefore it is constituted by a heart stimulator of the rate responsive type.

In general, the block 1 is connected to a catheter (lead) the tip C of which is inserted into the myocardium according to known criteria. The connection is achieved via input terminals 2 where an endocavity electrocardiological, ECG signal, is present (according to widely known criteria) in 2a, and a natural heart acceleration signal NHA (t) is present in 2b, detected, for example, with a sensor such as that described in EP-A-0 515 319.

These signals are usually referred to a second, reference electrode constituted, for example, by a ring positioned on the same catheter or by the metal container of the therapeutic device D, usually disposed under the skin.

In Figure 2 the terminals 2a and 2b lead to an analogue-to-digital converter interface 3.

The interface 3 transfers the signals provided by the electrode 2a and the accelerometer 2b, converted into digital form, to the input of a microprocessor 4. This latter constitutes, together with respective memories, for example of the ROM 5 and RAM 6 type, a control and processing system generally indicated 7 with an associated internal clock 8 as well as an output interface module 9 intended to control the therapeutic device D, via respective output terminals 10.

The bi-directional telemetry device 11 comprises a reception/transmission interface 12 connected to the microprocessor 4 as well as a radio frequency receiver-transmitter 13 connected in bi-directional relationship with the interface 12 leading to a receiver/transmitter antenna 14.

The processing and control circuit 7 supervises all the operating phases of the device and processes the data coming from the adjacent interfaces. To this end the ROM memory 5 preferably functions as a programme memory whilst the RAM memory 6 preferably functions as a processor memory and maintains the programme data saved by the microprocessor 4 in dependence on the processing performed.

The receiver-transmitter 11 exchanges information between an external programmer device 15 and the control system 7. In particular it serves for reception of externally programmed parameters and transmission of values memorised in the RAM memory 6. Typically, the data transmitted relate to parameters processed by the system after measurement has been effected or act to control the correct dialogue between the external programmer and the implanted device. The transmission and reception of data between the implantable device and the external programmer takes place as already mentioned , at radio frequency. All this is according to technical characteristics well known to those skilled in the art.

The internal clock 8, which is also programmable, is able to provide the processing system with the timing reference essential for a correct time base to which all the measurements are referred.

The processing operations performed by the system 7 on the signals coming from the terminals 2a and 2b have in the first place the purpose of analyzing the electrocardiographic signal (terminal 2a) for identifying electrocardiologicaily significant phases and/or events such as, for example, the QRS and/or the T-wave, recognising them both by amplitude and duration; other parameters are likewise detected such as the beat period and frequency.

All this takes place according to criteria known per se.

Starting from the analysis of the ECG signal the system 7 acts on the NHA(t) signal isolating within the ambit thereof determined temporal phases (segments) constituting, for example, phases of important haemodynamic significance.

Specifically, the control and processing system 7 is able to identify (isolate) those signals extensively referred to in the introductory portion of the description.

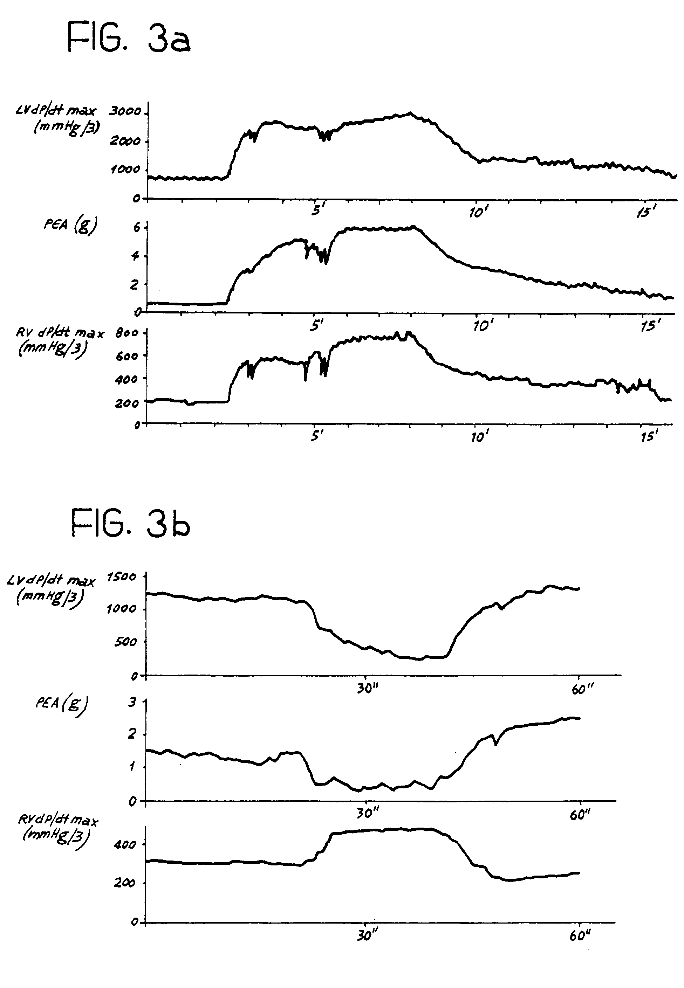

Figs. 3a and 3b show data taken during experiments with an anesthetized sheep. Fig. 3a shows RV dP/dt maximum and LV dP/dt maximum measured over a period of 15 minutes during positive inotropic stimulation with a dobutamine infusion. PEA was measured with a sensor in the apex of the right ventricle over the same period of time. Fig. 3a shows a direct correlation between PEA, RV dP/dt maximum and LV dP/dt maximum over the 15 minute time period under normal heart conditions.

During the same experiment a condition of acute RV pressure overload and inadequate LV filling was created by occluding the pulmonary artery with a balloon. Fig. 3b shows RV dP/dt maximum and LV dP/dt maximum measured over a period of 60 seconds during the occlusion. PEA was again measured over the same 60 second period. The data in Fig. 3b shows a direct correlation between LV dP/dt maximum and PEA and demonstrates the dominance of LV contractility in modulating the PEA changes.

The data supports the conclusion that PEA measured from a sensor in the right ventricle provides an accurate indication of the contractile function of the whole heart, even under conditions where RV dP/dt maximum and LV dP/dt maximum do not correlate.

It has also been shown experimentally that PEA shows considerable correlation to heart rate under adrenergic stimulation such as during physical and mental stress tests. However, it has also been shown experimentally that PEA is not significantly dependent on heart rate variations induced by pacing at rest. This data supports the use of PEA as an effective physiological parameter upon which a rate responsive pacing system can be based.

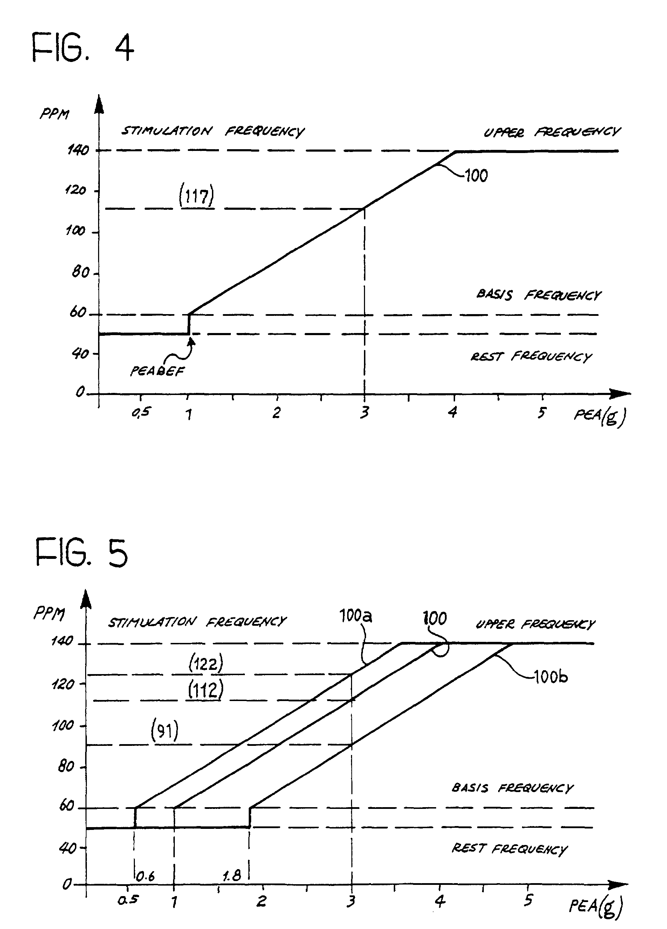

Fig. 4 illustrates the graph of an algorithm based on the use of PEA to adjust the pacing rate of a rate responsive pacemaker similar to that discussed with respect to Figs. 1 and 2. A PEA reference value (designated PEAREF in the Figures)is selected to reflect the basal condition of the heart. PEAREF is obtained by measuring PEA on a cycle-by-cycle basis and using a mobile averaging process with a long time constant. Thus, PEAREF is a floating value updated on a continuing basis based upon the average PEA value over a long period which may be from one hour or less to 48 hours or more. By selecting a sufficiently long time period over which to average PEA, the PEAREF value provides an accurate PEA basal value of the contractile function of the heart unaffected by short episodes of physical activity and/or mental stress. The system is self adjusting such that the value of PEAREF will change to accommodate long term changes in the basal state of the heart.

As seen in Fig. 4 the pacing rate adjusts from a lower limit or rest rate to an upper rate limit. In this case the rest rate is selected as 50 pulses per minute (ppm) and the upper rate is 140 ppm. These limits may be fully programmable so that they may be adjusted according to the needs or conditions of the patient. The basic rate is indicative of the value of the pacing rate under basal conditions, i.e. free of stress or physical activity. The rest rate may be programmed as an option to allow a lower heart rate at that point where the current value of PEA is just below PEAREF. In this example, the basic rate is set at 10 ppm over the rest rate. The rest rate is believed to be desirable to enable the heart rate to adjust to a lower value than the normal basal rate (basic rate) such as during periods of sleep. It should be noted however, that the difference between the basic rate and the rest rate can be programmed from zero to greater than 10 ppm.

The slope 100 between the basic rate and the upper rate serves as the rate response curve establishing the relationship between currently measured PEA and pacing rate. Although shown as being linear, slope 100 can be programmed to effect any desired nonlinear relationship between pacing rate and PEA.

The pacing rate is calculated cycle-by-cycle as a function of the difference between the currently measured PEA and PEAREF. For example, as illustrated in Fig. 4, if the current PEA is constant and equal to 3g, the pacing rate will be approximately 112 ppm.

Fig. 5 illustrates the effect of a change in PEAREF on the pacing rate. If PEAREF decreases from 1g to 0.6g slope 100 shifts to the left to the position of slope 100a. In that case a currently measured PEA of 3g results in a pacing rate of approximately 122 ppm. If the PEAREF increases to approximately 1.8g slope 100 shifts to the right to the position of slope 100b. At that position the pacing rate at a currently measured PEA of 3g is approximately 91 ppm. It is important to note the differential nature of the algorithm upon which the pacing rate is based. Thus, for example, an increase in PEA of 1g, i.e. from 2g to 3g, will cause the pacing rate to increase by the same amount whether the slope is in the position of slope 100a, 100 or 100b.

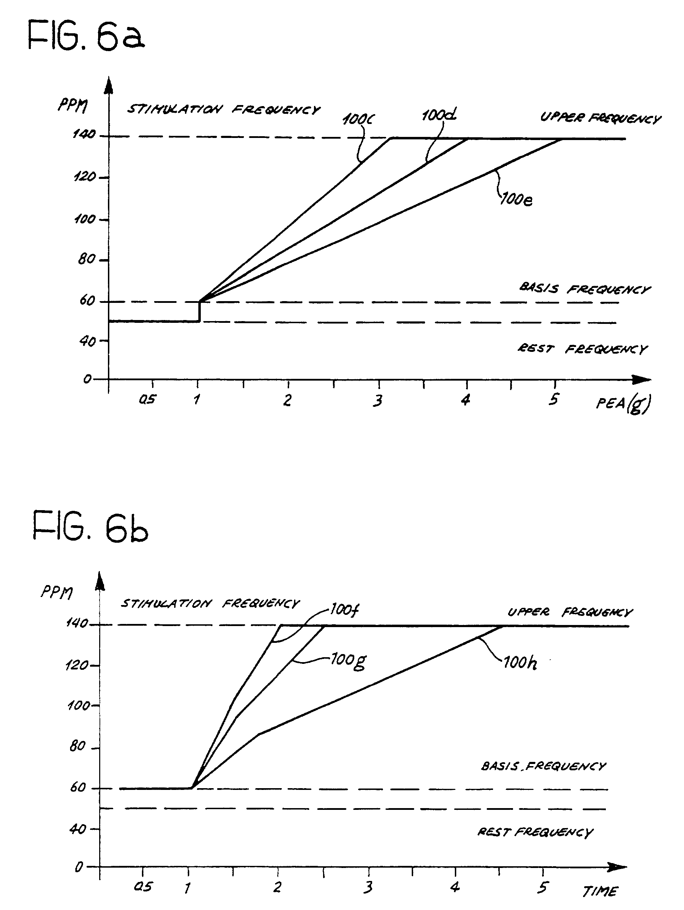

As seen in Figs. 6a and 6b, the rate response curve may be programmed to have a higher or lower response depending on the needs of the patient. Fig. 6a illustrates a group of response curves 100c, 100d and 100e. Curve 100c has a higher pacing rate response than curve 100d, which in turn has a higher response than curve 100e. Fig.6b illustrates a group of nonlinear rate response curves 100f, 100g and 100h having response rates varying from very fast (100f) to medium (100g) to slow (100h). Figs. 6a and 6b show just a few examples of the way the pacemaker may be programmed to achieve the particular response desired for a patient.

In the invention it is believed that PEA will serve as an effective parameter with which to control the atrioventricular delay (AV delay) for those patients suffering from some form of heart conduction disease which may be associated with cardiomyopathy. It is known that cardiac output (CO), heart rate (HR) and stroke volume (SV) are related according to the following formula:

$$CO = HR \times SV$$

Since stroke volume is dependent in part upon AV delay it is clear that cardiac output will be optimized when the AV delay is set to allow optimal time for filling of the ventricles.

It is believed that there is a correlation between AV delay and PEA. Therefore, an algorithm based upon the relationship between AV delay and PEA is used to set AV delay between upper and lower programmable limits. The algorithm is used to optimize AV delay as a function of the currently measured PEA. This feature can be used alone or in combination with the rate responsive feature discussed above. Thus, the pacemaker is able to adjust pacing rate and/or AV delay to provide a system which optimizes cardiac output according to a patient's needs.

**Claims**

1. A stimulator comprising

   a natural heart acceleration sensor (1) ;
   a signal generator coupled to the sensor to provide a signal representative of the natural heart acceleration (NHA);
   a processor (4) connected to receive the signal for determining a value (PEA) indicative of a characteristic of the signal during a selected time segment, the processor being operative to select a pacing rate;
   a pulse generator (D) responsive to the selected pacing rate to generate stimulation pulses corresponding to the selected pacing rate; and
   at least one electrode (C) for applying the stimulation pulses to at least one chamber of a patient's heart,

   **characterised in that**

   the processor (4) is connected for calculating the difference between the determined value and a reference value (PEAREF) and operative to select the pacing rate based upon the difference between the determined value and the reference value,
   the reference value (PEAREF) is a dynamic value calculated by the processor as the average of the determined value (PEA) over a predetermined time interval,
   the selected time segment is a phase of a cardiac cycle, wherein the phase of the cardiac cycle is the isovolumetric contraction phase, and the characteristic of the signal comprises the peak-to-peak value of the natural heart acceleration (PEA),

   wherein the processor (4) is operative to select a pacing rate and an AV delay based upon the determined value.

2. The stimulator of claim 1 wherein the predetermined time interval is in the range of between about one hour and about 48 hours.

3. The stimulator of claim 1 further comprising an ECG sensor to generate a signal representative of the ECG and wherein the time segment of the natural heart acceleration signal which is selected corresponds to a predetermined portion of the ECG signal.

4. The stimulator of claim 3 wherein the predetermined portion of the ECG signal comprises the isovolumetric contraction phase of the cardiac cycle.

## Patentansprüche

1. Ein Stimulator mit

einem Sensor (1) zur Erfassung der natürlichen Herzbeschleunigung;

einem Signalerzeuger, der mit dem Sensor gekoppelt ist, um ein Signal zu liefern, das repräsentativ für die natürliche Herzbeschleunigung (NHA) ist;

einem Prozessor (4), der angeschlossen ist, um das Signal zu erhalten und einen Wert (PEA) zu bestimmen, der indikativ für eine Charakteristik des Signals während eines ausgewählten Zeitsegments ist, wobei der Prozessor wirksam ist, um eine Schrittgeschwindigkeit zu wählen;

einem Impulserzeuger, der auf die gewählte Schrittgeschwindigkeit reagiert, um Stimulationsimpulse zu erzeugen, die der gewählten Schrittgeschwindigkeit entsprechen; und

wenigstens einer Elektrode (C), um die Stimulationsimpulse wenigstens einer Herzkammer eines Patienten zuzuführen,

**dadurch gekennzeichnet, daß**

der Prozessor (4) angeschlossen ist, um den Unterschied zwischen dem bestimmten Wert und einem Referenzwert (PEAREF) zu berechnen, und wirksam ist, um die Schrittgeschwindigkeit basierend auf dem Unterschied zwischen dem bestimmten Wert und dem Referenzwert auszuwählen,

der Referenzwert (PEAREF) ein dynamischer Wert ist, der von dem Prozessor als der Durchschnitt des bestimmten Wertes (PEA) über ein vorgegebenes Zeitintervall berechnet wird;

das ausgewählte Zeitsegment eine Phase ein Herzzyklus ist, wobei die Phase des Herzzyklus die isovolumetrische Kontraktionsphase ist, und

die Charakteristik des Signals den Spitze-zu-Spitze-Wert der natürlichen Herzbeschleunigung (NHA) ist, wobei der Prozessor (4) wirksam ist, um die Schrittgeschwindigkeit und eine AV-Verzögerung basierend auf dem bestimmten Wert auszuwählen.

2. Der Stimulator von Anspruch 1, worin das vorgegebene Zeitintervall in dem Bereich von zwischen einer Stunde und etwa 48 Stunden liegt.

3. Der Stimulator von Anspruch 1, mit weiterhin einem EKG-Sensor, um ein Signal zu erzeugen, das repräsentativ für das EKG ist, wobei das Zeitsegment des natürlichen Herzbeschleunigungssignals, welches ausgewählt wird, einem vorgegebenen Bereich des EKG-Signals entspricht.

4. Der Stimulator nach Anspruch 3, worin der vorgegebene Bereich des EKG-Signals die isovolumetrische Kontraktionsphase des Herzzyklus enthält.

## Revendications

1. Stimulateur comprenant :

un capteur de l'accélération naturelle du coeur (1),
un générateur de signal couplé au capteur pour fournir un signal représentatif de l'accélération naturelle du coeur (NHA) ;
un processeur (4) relié pour recevoir le signal pour déterminer une valeur (PEA) indicative d'une caractéristique du signal pendant un segment de temps sélectionné, le processeur étant opérationnel pour sélectionner une vitesse de stimulation ;
un générateur d'impulsion (D) sensible à la vitesse de stimulation sélectionnée pour générer les impulsions de stimulation correspondant à la vitesse de stimulation sélectionnée ; et
au moins une électrode (C) pour appliquer les impulsions de stimulation à au moins une chambre du coeur d'un patient,

**caractérisé en ce que** le processeur (4) est relié pour calculer la différence entre la valeur déterminée et une valeur de référence (PEAREF) et est opérationnel pour sélectionner la vitesse de stimulation basée sur la différence entre la valeur déterminée et la valeur de référence,

la valeur de référence (PEAREF) est une valeur dynamique calculée par le processeur comme la moyenne de la valeur déterminée (PEA) sur un intervalle de temps prédéterminé,

le segment de temps sélectionné est une phase d'un cycle cardiaque, dans lequel la phase du cycle cardiaque est la phase de contraction isovolumétrique, et

la caractéristique du signal comprend la valeur crête à crête de l'accélération naturelle du coeur (PEA), dans lequel le processeur (4) est opérationnel pour sélectionner une vitesse de stimulation et un retard AV basé sur la valeur déterminée.

2. Stimulateur selon la revendication 1, dans lequel l'intervalle de temps prédéterminé se situe dans la gamme comprise entre environ une heure et environ 48 heures.

3. Stimulateur selon la revendication 1 comprenant un capteur ECG pour générer un signal représentatif du ECG et dans lequel le segment de temps du signal d'accélération naturelle du coeur qui est sélectionné, correspond à une partie prédéterminés du signal ECG.

4. Stimulateur selon la revendication 3 dans lequel la partie prédéterminés du signal ECG comprend la phase de contraction isovolumétrique du cycle cardiaque.

# FIG. 1

# FIG. 2

# FIG. 3a

# FIG. 3b

# FIG. 4

# FIG. 5

## FIG. 6a

PPM — STIMULATION FREQUENCY — 100c — 100d — UPPER FREQUENCY
140
120
100
80 — 100e
60 — BASIS FREQUENCY
40 — REST FREQUENCY
0 — 0.5 — 1 — 2 — 3 — 4 — 5 — PEA(g)

## FIG. 6b

PPM — STIMULATION FREQUENCY — 100f — UPPER FREQUENCY
140
120 — 100g
100
80 — 100h
60 — BASIS FREQUENCY
40 — REST FREQUENCY
0 — 0.5 — 1 — 2 — 3 — 4 — 5 — TIME